# EUROPEAN PATENT APPLICATION

(11) **EP 1 531 059 A1**
(43) Date of publication of application: **18.05.2005**
(21) Application number: 04257011.9
(22) Date of filing: 11.11.2004
(51) Int. Cl.: B41M 7/00, B41M 1/30, A61F 13/02

(54) **Process for printing on a continuous strip of flexible film, an adhesive bandage obtained by said process, and a strip of decorated flexible film**

(30) Priority: 12.11.2003 BR 0305610
(71) Applicant: Johnson & Johnson Industrial Ltda., Sao Jose dos Campos, SP (BR)
(72) Inventor: Spinardi Jr., Walter, Sao Jose dos Campos, SP (BR)
(74) Representative: Mercer, Christopher Paul

(57) **Abstract**

A process for printing on a continuous strip (10) of flexible film and a strip of decorative flexible film, such as a plastic film used in the formation of adhesive bandages, comprising the steps of:
- passing the continuous strip through a plurality of printing stations (5), which are arranged in series and intercalated by drying stations (11), each of the printing stations (5) printing, with solvent or water based ink, along the width of an upper face of the continuous strip (10), a plurality of predetermined respective image portions;
- passing the continuous strip (10) through a varnishing station (20), in order to cover the upper face of the continuous strip (10) and the image portions printed thereon with a photopolymerizable varnish layer;
- passing the varnished continuous strip through a photopolymerization station (30), in which the varnish is photopolymerized preferably by ultraviolet (UV) radiation; and
- conducting the continuous strip (10) to a subsequent step of any process for forming an adhesive bandage with said continuous strip (10).

## Description

### Field of the Invention

The present invention refers to printing processes, preferably of the flexographic type, to be effected on a continuous strip of flexible film, for example a plastic flexible film for the formation of adhesive bandages, by using solvent based inks and/or varnishes, and photopolymerizable inks and/or varnishes. The present invention further refers to the continuous strip of decorative flexible film obtained by said process.

### Prior Art

The known adhesive bandages to be applied to small skin wounds, such as light cuts, abrasions, scratches, etc., are generally formed of a thin and flexible basic film, for example a plastic film, having a non-adhesive external face for handling the bandage, and an internal face receiving an adhesive layer and securing, in a median region, an absorbent pad that will contact the skin in the wounded region, and in its regions not covered by the absorbent pad, respective protecting strips which are easily released from the basic film when the bandage is applied to the user's skin.

In the adhesive bandages directed to children, it is common to provide the non-adhesive external face of the basic film with imprints of decorative images or characters, with the purpose of making the product more attractive to the consumer, minimizing its usual unpleasant appearance.

A known solution for the problem of lack of attractiveness that the conventional adhesive bandages exert on children is described in US patent 4,094,316 and comprises the provision of an adhesive applique, containing a decorative imprint on its lower face which is releasably affixed to the non-adhesive external face of the basic film. Anyhow, the adhesive applique is also obtained from a continuous strip of a flexible film to have its non-adherent external face provided with a decorative imprint.

Printing of the decorative characters on a continuous strip of flexible film, generally a plastic film to be used for example, as a basic film in the formation of adhesive bandages, is usually accomplished by the flexographic processes, using equipments which differ from each other as a function of the width of the continuous strip onto which the printing will be made. In the case of the continuous strips with a width, for example of 700mm, there is little or no longitudinal deformation of the continuous strip during its passage through the different printing phases. However, in the narrower continuous strips, a degree of longitudinal deformation usually occurs of the continuous strip, which is generally unacceptable because it causes loss of the dimensional and esthetic standard of the decorative characters.

When the objective is the provision of decorative imprints on a continuous strip of a flexible film presenting a width, for instance of 700mm, solvent or water based inks and/or varnishes are used, which are dried by application of heat.

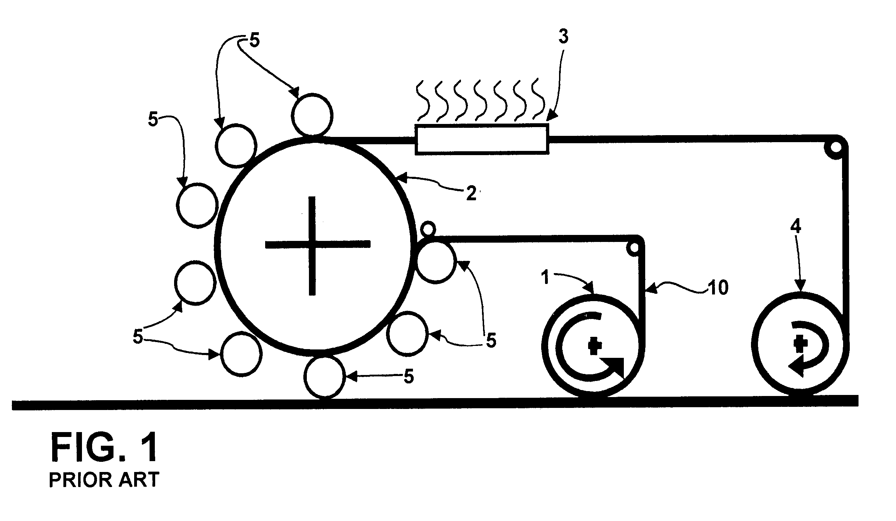

Figure 1 is a simplified flowchart of the process for printing decorative characters on a continuous strip of flexible film with a width, for example of 700 mm. The continuous strip 10 of flexible film is continuously withdrawn from a feed reel 1, of any adequate construction, and conducted to a rotary central drum 2, also known in the art and around which the continuous strip is seated along a circumferential extension of about 180° or more, before being directed to a drying station 3, in which the solvent or water is evaporated by application of heat and wherefrom it leaves toward a storage reel 4. During its passage over the rotary central drum 2, the continuous strip 10 is sequentially submitted to different ink and/or varnish printing stations 5, intercalated by drying stations disposed around the rotary central drum 2, each of the printing stations being responsible for applying a determined color of ink and/or varnish to the continuous strip 10 seated on the rotary central drum 2.

The solvent or water based inks and/or varnishes utilized in the process mentioned above have low cost and are associated with high productivity, resulting from the high printing speeds and the large number of repetitive decorative images, which are simultaneously produced along the large width of the continuous strip 10. Thus, an end product (decorated flexible film) is obtained, of low cost and good printing quality. Since the continuous strip 10 presents a large width and is only seated around the rotary central drum 2 during the printing process, there occurs no stretching or longitudinal elongation of the continuous strip 10 which is sufficient to impair the registration integrity of the decorative images that are printed within the predetermined dimensional standard.

However, the end product obtained by the above-mentioned process using solvent or water based inks and/or varnishes does not present a satisfactory bright appearance.

The process for printing on continuous strips of flexible films using photopolymerizable inks and/or varnishes is also known. Such process however, as a function of its characteristics, is not economically viable for printing on strips of large width, for example superior to 254 mm.

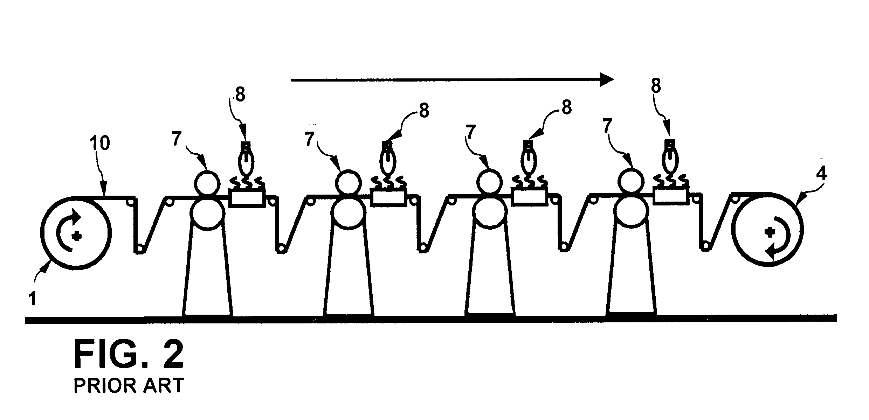

Figure 2 of the enclosed drawings is a simplified flowchart of the process for printing decorative characters with photopolymerizable ink and/or varnish on continuous strips 10, which are considered narrow and in which the relative width reduction is already a factor of productivity reduction.

In the process above, the continuous strip 10 (of smaller width) is continuously withdrawn from a feed reel 1, of any adequate construction, and supplied through a plurality of printing stations 7 arranged in series and through which the continuous print 10 is pulled, each printing station 7 providing the application of an ink and/or varnish to the decorative image printed on the continuous strip 10. Immediately after leaving each printing station 7, the continuous strip 10 has the recently applied ink and/or varnish submitted to a photopolymerization promoted by a respective photopolymerization station 8, which is generally defined by one or more preferably ultraviolet (UV) lamps. After leaving the last photopolymerization station 8, the continuous strip 10 is wound around a storage reel 4.

The fact of the continuous strip 10 being pulled along the plurality of printing stations 7 arranged in series makes the continuous strip 10 be submitted to longitudinal efforts, which are sufficiently strong to produce the undue stretching of the flexible film, making impossible to maintain the registration integrity of the decorative images within the dimensional standard predetermined for the end product. A known solution for the deficiency above is to support the continuous strip 10 of flexible film with a paper strip carrier (siliconized for example) already containing the adhesive required for the formation of the bandage. This artifice eliminates the problem of deviations in the registration of the printed images, but imposes a considerable increase on the final cost of the product.

The process for printing, by using photopolymerizable inks and/or varnishes on the continuous strip 10 of flexible film, with a relatively reduced width and supported by a paper strip carrier, allows obtaining an end product with high printing quality and registration of images, and also having a bright appearance, resulting from the photopolymerizable inks and/or varnishes, which is not attained with the solvent or water based inks and/or varnishes. However, the production cost related to this process is high, as a function of the inks and/or varnishes utilized, and of the lower productivity of the repetitive images printed on a continuous strip of smaller width.

### Objects of the Invention

By reason of the deficiencies mentioned above related to the known prior art process for printing on continuous strips of flexible film, it is an object of the present invention to provide a process of the type considered herein, which allows obtaining decorative imprints on continuous strips of flexible film used in the production of adhesive bandages with characteristics, such as high printing quality, high image registration, desired bright appearance and low cost.

It is a further object of the present invention to provide a continuous strip of decorative flexible film obtained in accordance with the above-mentioned process.

### Summary of the Invention

According to a first aspect of the present invention, the present process for printing on a continuous strip of flexible film, such as a plastic film used in the formation of adhesive bandages, comprises the steps of:
- passing continuously and progressively the continuous strip through a plurality of ink and/or varnish printing stations, which are arranged in series and intercalated by drying stations, each of said printing stations printing, with solvent or water based ink and/or varnish along the width of an upper face of the continuous strip, a plurality of predetermined respective image portions;
- passing continuously and progressively the continuous strip through a varnishing station, in order to cover the upper face of the continuous strip and the image portions printed thereon with a photopolymerizable varnish layer;
- passing the varnished continuous strip through a photopolymerization station in which the varnish is preferably photopolymerized by ultraviolet (UV) radiation; and
- conducting the continuous strip to a subsequent step of any process for forming an adhesive bandage with said continuous strip.

The process described above allows producing a continuous strip of flexible film, presenting a width for example of 700 mm, and carrying image portions which are printed with low cost inks and/or varnishes, said images presenting high printing quality and high image registration with an excellent bright appearance.

The process mentioned above allows obtaining a continuous strip of decorated flexible film presenting a face which carries, along its width, a plurality of decorative image portions that are printed on a continuous strip, by using solvent or water based inks and a photopolymerizable varnish layer applied to said face of the continuous strip, covering the latter and the decorative image portions printed thereon.

### Brief Description of the Drawings

The invention will be described below, with reference to the enclosed drawings, given by way of example of a form of carrying out the invention and in which:
Figure 1 is a simplified flowchart of a process for printing decorative images on a continuous strip of flexible film, with a width for example of 700 mm, and by using solvent or water based inks and/or varnishes;
Figure 2 is a simplified flowchart of a process for printing decorative images on a continuous strip of flexible film, with a width for example of 254 mm, and by using inks and/or varnishes that can be dried preferably by ultraviolet (UV) rays;
Figure 3 is a simplified flowchart of the process of the present invention, in which the printing of solvent or water based inks and/or varnishes is effected on the continuous strip, which is subsequently coated with a photopolymerizable varnish layer;
Figure 4 is a simplified flowchart of the printing station of the flexographic process of the present invention; and
Figure 5 is a simplified flowchart of the varnishing station of the flexographic process of the present invention.

### Detailed Description of the Invention

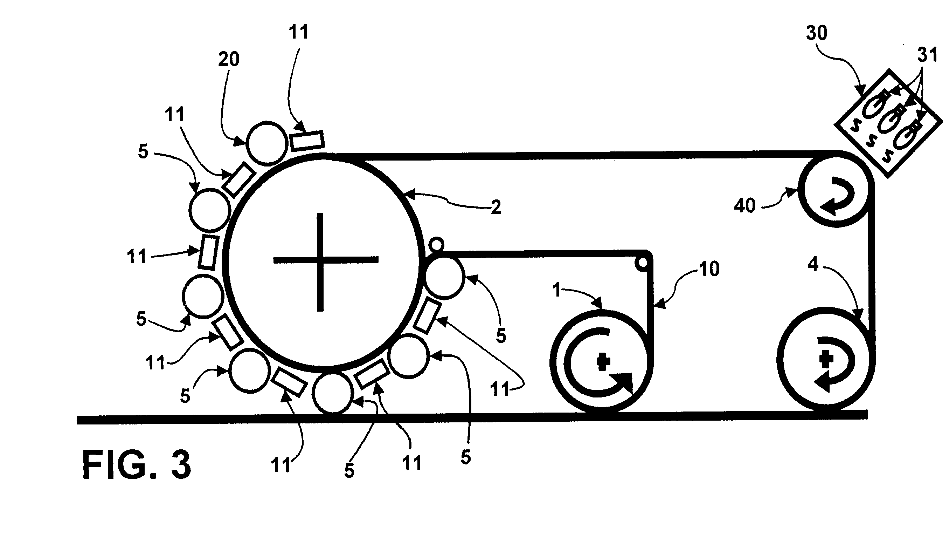

The process for printing on a continuous strip of the present invention can be of the rotogravure, letterpress, or any other type of applicable printing process known in the state of the art. Preferably, the present process for printing on a continuous strip is of the flexographic type, as illustrated in figure 3.

Such printing process is performed on a continuous strip 10 of flexible film, presenting a width for example of 700 mm and which can be made of different materials, such as polymeric plastic films selected from the group consisting of polyethylene, polypropylene, polyester, polyurethane, ethylvinyl acetate (EVA), polyvinyl chloride (PVC) and other similar films and/or mixtures thereof.

The continuous film 10, which is generally wound on a feed reel 1, and continuously withdrawn therefrom and conducted to a rotary central drum 2, which is well known in the technique of flexographic printings on flexible films, said rotary central drum 2 presenting a cylindrical lateral surface and a generally horizontal axis. The continuous strip 10 is continuously withdrawn from the feed reel 1, with a lower face seated on a certain circumferential extension, which is generally superior to 180°, of the cylindrical surface of the rotary central drum 2, and thence conducted to a storage reel 4 where it is rewound.

While passing on the cylindrical surface of the rotary central drum 2, the continuous strip 10 is continuously and progressively submitted to a plurality of ink and/or varnish printing stations 5 arranged in series and which are intercalated, around the rotary central drum 2, by drying stations 11, each of the ink and/or varnish printing stations 5 printing, with solvent or water based ink and/or varnish and along the width of an upper face of the continuous strip 10, a plurality of predetermined respective image portions (not illustrated).

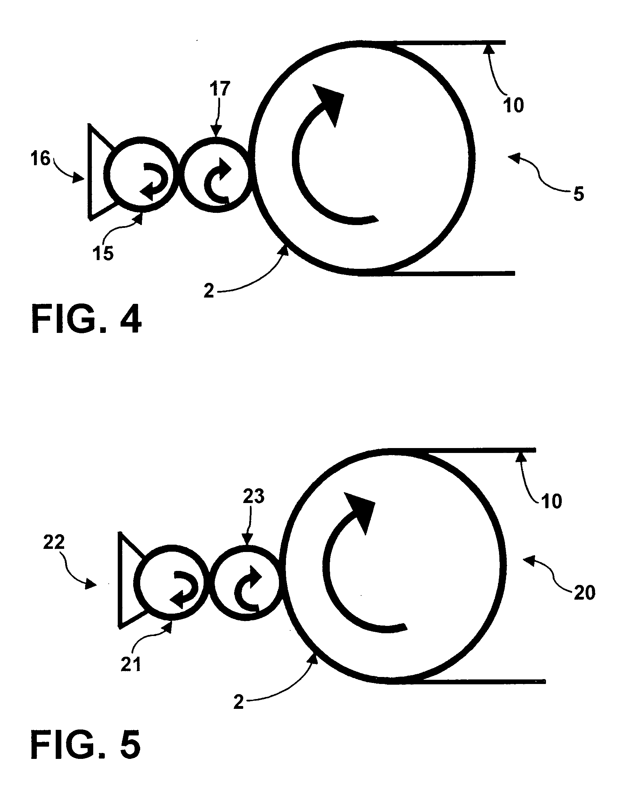

As illustrated in figure 4, the ink and/or varnish printing stations 5 comprise, each one, a flexographic printing system comprising an anilox roll 15, provided with a doctor blade system 16 and which is responsible for the quantity of ink and/or varnish to be applied onto the continuous strip 10, and a roll coated with a cliché 17 containing the image to be printed. Each ink and/or varnish printing station 5 prints, on the width of the upper face of the continuous strip 10, a plurality of image portions defined in a determined color and design, the set of imprints effected by the sequence of ink and/or varnish printing stations 5 conducting to the formation of the decorative images to be printed on said continuous strip 10.

The present process further comprises the step of drying the ink and/or varnish printed on the continuous strip 10, making the latter pass through drying stations 11, which are provided immediately after each ink and/or varnish printing station 5, the solvent or the water of the printing ink and/or the varnish being evaporated by application of heat in this drying station 11.

Upon completion of the steps of printing ink and/or varnish and of drying along said circumferential extension of the cylindrical surface of the rotation central drum 2, the continuous strip 10 is caused to pass continuously and progressively through a varnishing station 20, in which the upper face of the continuous strip 10 and the image portions printed thereon are coated with a varnish layer formulated with photopolymerizable resins. Such photopolymerizable resins can be photopolymerized by infrared rays, electron beam, preferably by ultraviolet (UV) rays. The varnishing station 20 can be constructed in any adequate manner known in the art, provided it can guarantee the uniform application of a thin varnish layer on the entire decorative upper face of the continuous strip 10 which leaves the rotary central drum 2 towards the storage reel 4.

Preferably, as illustrated in figure 5, the varnishing station 20 comprises an anilox roll 21 provided with a doctor blade system 22 and which is responsible for the quantity of varnish to be applied on the continuous strip 10. The varnishing station 20 also comprises a roll 23, which can be coated with a cliché, with the purpose of making applications on specific areas along the continuous strip 10. The roll 23 can also be coated with rubber for making applications on the whole area of the continuous strip 10.

As illustrated in figure 3, after leaving the varnishing station 20, the already varnished continuous strip 10 is made to pass through a photopolymerization station 30 in which the varnish is photopolymerized preferably by ultraviolet (UV) radiation. The decorated strip, presenting the desired qualities of brightness due to the application of the varnish layer on the decorative image portions, is conducted to a subsequent step of any process for forming an adhesive bandage with said continuous strip 10.

As illustrated in figure 3, the continuous strip 10 is preferably refrigerated while passing over a cooling roll 40, which allows maintaining the continuous strip 10 at a reduced temperature, in order to avoid its longitudinal deformation while being transferred from the feed reel 1 to the storage reel 4, passing through the different printing stations 5 and the varnishing station 20.

The ultraviolet (UV) radiation in the photopolymerization station 30 can be produced by different sources of adequate ultraviolet (UV) rays of known construction. In the illustrated example, the ultraviolet (UV) radiation is produced by a plurality of lamps 31, preferably of ultraviolet (UV) rays, which are positioned so as to cover the whole width of the continuous strip 10 and to produce the necessary radiation to effect the desired photopolymerization on the applied varnish, at a given processing speed of the continuous strip 10.

The process mentioned above allows forming a continuous strip of a decorated flexible film, presenting a face which carries, along the width of the continuous strip, a plurality of decorative image portions printed in solvent or water based ink and/or varnish, and a photopolymerizable varnish layer applied on said face of the continuous strip, covering the latter and the decorative image portions printed thereon.

Any varnish can be used in the present invention, provided it matches the material of the continuous strip 10 and of the ink and/or varnish printings applied thereon.

For carrying out the present process, the photopolymerization station 30 can be constructed with deflectors and quartz based means, for capturing and absorbing the infrared rays and which are designed to absorb the heat generated by the preferably UV lamps 31, thus preventing the heat from being transferred to the continuous strip 10 and causing deformations thereon.

The application of the varnish layer on the decorative image portions provided on the upper part of the continuous strip 10 allows obtaining an end product presenting a brightness rate equivalent to that obtained by the printing process using photopolymerizable inks and/or varnishes and which is about seven times higher than the brightness rate obtained by the conventional printing process using solvent or water based inks and/or varnishes. Furthermore, the cost of the product manufactured by the present process is approximately the same as that of the conventional process with low brightness, but which is about 40% less expensive than the printing process using photopolymerizable inks and/or varnishes.

## Claims

1. A process for printing on a continuous strip of flexible film, such as a plastic film used in the formation of adhesive bandages, **characterized in that** it comprises the steps of:
- passing continuously and progressively the continuous strip (10) through a plurality of ink and/or varnish printing stations, which are arranged in series and intercalated by drying stations, each of said printing stations printing, with solvent or water based ink and/or varnish, along the width of an upper face of the continuous strip, a plurality of predetermined respective image portions;
- passing continuously and progressively the continuous strip (10) through a varnishing station (20), in order to cover the upper face of the continuous strip (10) and the image portions printed thereon with a photopolymerizable varnish layer;
- passing the varnished continuous strip (10) through a photopolymerization station (30) in which the varnish is photopolymerized; and
- conducting the continuous strip (10) to a subsequent step of any process for forming a product with said continuous strip (10).

2. The process as set forth in claim 1, **characterized in that** the continuous strip (10) is refrigerated in a cooling station (40).

3. The process as set forth in claim 1, **characterized in that** the ink and/or vanish printing stations (5) are operatively associated with the cylindrical surface of a rotary central drum (2), with a not printed lower face of the continuous strip (10) being seated on a circumferential extension of said cylindrical surface during its passage through the ink and/or varnish printing stations (5).

4. The process as set forth in claim 3, **characterized in that** each ink and/or vanish printing station (5) comprises a flexographic printing system comprising an anilox roll (15) provided with a doctor blade system (16) and which is responsible for the quantity of ink and/or vanish to be applied on the continuous strip 10, and a roll (17) which is coated with a cliché containing the image to be printed.

5. The process as set forth in claim 1, **characterized in that** it comprises a photopolymerization station 30.

6. The process as set forth in claim 5, **characterized in that** the photopolymerization station (30) comprises ultraviolet (UV) radiation produced by at least one lamp.

7. A strip of decorated flexible film, **characterized in that** it presents a face carrying, along the width of the continuous strip (10), a plurality of decorative image portions printed with solvent or water based ink and/or varnish, and a photopolymerizable varnish layer, which is applied on said face of the continuous strip (10), covering the latter and the decorative image portions printed thereon.

8. The strip as set forth in claim 7, **characterized in that** it presents a width that is generally superior to about 700 mm.

9. The strip as set forth in claim 7, **characterized in that** it comprises a polymeric plastic flexible film selected from the group consisting of polyethylene, polypropylene, polyester, polyurethane, ethylvinyl acetate (EVA), polyvinyl chloride (PVC) and other similar films and/or mixtures thereof.

10. An adhesive bandage, **characterized in that** it is obtained by the process described in the previous claims.
